# EUROPÄISCHE PATENTSCHRIFT

(11) **EP 0 296 189 B1**
(45) Veröffentlichungstag und Bekanntmachung des Hinweises auf die Patenterteilung: **19.01.1994**
(21) Anmeldenummer: 88900012.1
(22) Anmeldetag: 02.11.1987
(51) Int. Cl.: A61B 8/06, A61K 49/00, G01L 11/00

(54) **ULTRASCHALL-MANOMETRIEVERFAHREN IN EINER FLÜSSIGKEIT MITTELS MIKROBLÄSCHEN**
PROCESS FOR ULTRASONIC PRESSURE MEASUREMENT IN A LIQUID USING MICRO-BUBBLES
PROCEDE MANOMETRIQUE PAR ULTRASONS DANS UN LIQUIDE AU MOYEN DE MICROBULLES

(30) Priorität: 05.11.1986 DE 3637926
(43) Veröffentlichungstag der Anmeldung: 28.12.1988
(73) Patentinhaber: SCHERING AKTIENGESELLSCHAFT, 13342 Berlin (DE)
(72) Erfinder: SCHLIEF, Reinhard, D-1000 Berlin 37 (DE); POLAND, Hans, D-1000 Berlin 49 (DE)
(86) Internationale Anmeldenummer: EP8700654
(87) Internationale Veröffentlichungsnummer: WO8803388

(56) Entgegenhaltungen:
- EP-A- 52 575
- EP-A- 72 330
- EP-A- 77 752
- EP-A- 122 624
- EP-A- 123 235
- EP-A- 131 540
- US-A- 3 640 271
- US-A- 4 265 251

## Beschreibung

Die Erfindung betrifft ein Ultraschall-Manometrieverfahren nach dem Oberbegriff des Anspruchs 1.

In der Zeitschrift "IEEE Transaction on Biomedical Engineering, BME-24, Nr. 2, März 1977" wird auf den Seiten 107 bis 110 ein nichtinvasives Verfahren zur Druckmessung im Herzen beschrieben, bei dem von der Resonanzstreuung des Ultraschalls durch Bläschen Gebrauch gemacht wird. In der Flüssigkeit, deren Druck gemessen werden soll, werden Bläschen gleichförmiger Größe eines Gases, wie beispielsweise Stickstoff oder eines Inertgases erzeugt. Diese Bläschen haben die gleiche Resonanzfrequenz. Es wurden breitbandige Ultraschallimpulse in die Flüssigkeit eingestrahlt und das Spektrum des durchgelassenen oder gestreuten Ultraschalls untersucht. Bei dieser Arbeitsweise wird von einer Theorie ausgegangen, der die Wechselwirkung von Ultraschall und Einzelbläsehen zu Grunde liegt. Es wurde die Resonanzfrequenzverschiebung bei Druckänderungen festgestellt. Bei diesem Verfahren wurde die Resonanzverschiebung geeicht. Auf diese Weise konnte die Druckänderung in einer Herzkammer ermittelt werden.

Diese Meßtechnik, die auf der Wechselwirkung von Ultraschall und Einzelbläschen beruht, hat sich nicht bewährt, weil die kleinen Einzelblasen eine zu kurze Lebensdauer haben und größere Blasen, insbesondere bei Messungen im Menschen, zu gefährlich sind. Größere Blasen können zu Embolien führen.

Die bei dem in der vorstehend zitierten Literaturstelle beschriebenen Verfahren verwendeten Bläschen sind wegen ihrer Größe nicht lungengängig. Diese Bläschen sind nach einer venösen Injektion im arteriellen Bereich nicht mehr vorhanden. Aus diesem Grunde können nach venöser Injektion bei dem bekannten Verfahren der diastolische und der systolische Druck nur im rechten Vorhof und in der rechten Kammer, nicht aber im linken Vorhof und in der linken Kammer gemessen werden.

In dieser Literaturstelle wird noch darauf hingewiesen, daß die Herstellung gleichförmiger Mikrobläschen außerordentlich schwierig ist.

Der Erfindung liegt die Aufgabe zu Grunde, ein Ultraschall-Manometrieverfahren in einer Flüssigkeit zur Verfügung zu stellen, mit dem eindeutige und reproduzierbare Messungen sowohl von Druckänderungen als auch von Druckabsolutwerten in Röhrensystemen, insbesondere an beliebigen Stellen im menschlichen Körper, hier insbesondere an beliebigen Stellen im Herzen, durchgeführt werden können. Ausgehend von einem Verfahren der eingangs genannten Art, wird diese Aufgabe jeweils erfindungsgemäß aufgrund des Kennzeichens von Anspruch 1, gelöst.

Mit Vorteil werden bei dem erfindungsgemäßen Verfahren demnach keine einzelnen Gasbläschen, sondern ein Ensemble, das Mikrobläschen, die stabilisiert sind, enthält, verwendet. Es wird in der Flüssigkeit ein wolkenartiges Gebilde aus Mikrobläschen erzeugt. Dabei haben die stabilisierten Mikrobläschen eine Lebensdauer, die weit über der physikalisch für eine reine Flüssigkeit berechneten liegt. Das Verhalten eines solchen Mikrobläschenensembles ist nicht mehr mittels bekannter Resonanz- und Streumodelle von Einzelbläschen zu beschreiben. Es werden wesentlich größere, reproduzierbare Nutzsignale erzeugt. Dadurch kann eine eindeutige und reproduzierbare Messung durchgeführt werden, was bisher bei Einzelblasen nicht möglich war.

Ferner können mit Vorteil Mikrobläschen mit einem standardisierten, d. h. bekannten und reproduzierbaren Spektrum der Resonanzabsorption verwendet werden. Mittels der empfangenen Ultraschallimpulse kann die spektrale Absorption zur Feststellung des Absolutwertes des momentanen Flüssigkeitsdruckes ermittelt werden. Bei Verwendung von Mikrobläschen mit bekanntem oder standardisiertem Resonanzspektrum können mit Vorteil Ultraschallimpulse mit einem vorbestimmten Frequenzbereich eingestrahlt werden. Zur Auswertung können Frequenzbereiche registriert werden, die außerhalb des Frequenzbereichs der eingestrahlten Ultraschallimpulse liegen.

Zur Ausschaltung von Fehlersignalen können mit Vorteil Ultraschallimpulse eingestrahlt werden, deren Frequenzen in einem vorbestimmten Verhältnis zu den Resonanzfrequenzen der Mikrobläschen stehen. Dabei können insbesondere n-te harmonische oder subharmonische Frequenzen bezüglich der Resonanzfrequenzen verwendet werden. Mit Vorteil können bei diesen Verfahren Amplitudenspektren im Frequenzbereich oberhalb der Eigenfrequenzen der Mikrobläschen ausgewertet werden.

Es kann vorteilhaft sein, Mikrobläschen mit Durchmessern im Bereich von 0,1 bis 500 um zu verwenden. Dabei können Ultraschallfrequenzen mit Frequenzen im Bereich von 800 KHz bis 5 MHz eingestrahlt werden.

Durch die Erfindung wird ein Verfahren geschaffen, bei dem die Frequenzänderungen von Ultraschallsignalen berücksichtigt werden, die in Wechselwirkung mit Mikrobläschen in einer Flüssigkeit getreten sind.

Durch die Erfindung werden ferner Mittel zur Blutdruckmessung bereitgestellt.

Ein bevorzugtes Mittel wird dadurch gebildet, daß in einer Flüssigkeit Feststoffpartikel, die frei von Mikrobläschen sind und vorwiegend Partikel enthalten, die eine Anzahl Gas gefüllter Hohlräume aufweisen, die mit der Oberfläche in Verbindung stehen und eine Anzahl Kerne für eine Mikrobläschenbildung, insbesondere fein zerteilte Glucose, Laktoglucose, Maltose oder Salz mit Partikelgrößen im Bereich von 1 bis 5 µm gelöst werden, wobei das Verhältnis der Maße der Partikel zum Gasvolumen der Hohlräume so gewählt wird, daß dieses ausreicht, um die Flüssigkeit, in der die Feststoffpartikel gelöst sind, in dem Bereich, der die Mikrobläschen umgibt, bezüglich des Gases in den Hohlräumen zu übersättigen.

Eine injizierbare Suspension kann eine wäßrige Trägerflüssigkeit aufweisen, deren Viskosität wesentlich größer als die von Wasser ist. Wenn dieses Mittel zur Blutdruckmessung mittels des Ultraschall-Manometrieverfahrens verwendet wird, können mit Vorteil die Blutgefäße vollständig gefüllt werden. Die mit dem Mittel erzeugten, Mikrobläschen passieren auch Kapillaren der Lungen, wodurch eine intraarterielle Druckmessung nach peripher-venöser Injektion prinzipiell an nahezu jeder beliebigen Stelle des Blutgefäßsystems möglich wird.

In vorteilhafter Weise kann ein Mittel zur Blutdruckmessung mittels des Ultraschall-Manometrieverfahrens verwendet werden, welches Mikropartikel der Mischung von einer halbfesten oder flüssigen grenzflächenaktiven Substanz mit einem nicht-grenzflächenaktiven Feststoff in einem flüssigen Träger enthält.

Derartige Mittel, die erfindungsgemäß zur Blutdruckmessung verwendet werden können, werden in der EP-OS 123 235 beschrieben.

Mit Vorteil kann das Mittel Mikropartikel enthalten, die als halbfeste oder flüssige grenzflächenaktive Substanz Lecithine, Polyoxyethylenfettsäureester, Glycerinpolyethylenglykolrizinoleat, Polyoxyethylenpolyoxypropylen-Polymere, Saccharoseester, Xyloglyceride, ungesättigte (C₄-C₂₀)-Fettalkohole, ungesättigte (C₄-C₂₀)-Fettsäuren, Mono-, Di- und Triglyceride, Fettsäureester als Mikropartikel in einer Menge von 0,01 bis 10 Gewichtsprozent enthalten. Ferner ist es möglich, daß das Mittel Mikropartikel enthält, die als halbfesten oder flüssigen grenzflächenaktiven Stoff Butylstearat, Sojaölsaccharoseglycerid oder Polyethylenglykolsorbitanmonostearat in einer Konzentration von 0,01 bis 5 Gewichtsprozent, vorzugsweise 0,04 bis 1 Gewichtsprozent, enthalten. Als nicht-grenzflächenaktive Feststoffe kann das Mittel Cyclodextrine, Monosaccharide, Disaccharide, Trisaccharide, Polyole oder anorganische oder organische Salze mit einer Konzentration von 5 bis 50 Gewichtsprozent enthalten. Das Mittel kann ferner Mikropartikel enthalten, die als nicht-grenzflächenaktiven Feststoff Galaktose, Lactose oder a-Cyclodextrin in einer Konzentration von 5 bis 50 Gewichtsprozent, vorzugsweise von 9 bis 40 Gewichtsprozent, enthalten.

Mit besonderem Vorteil kann das Mittel als physiologisch verträglichen flüssigen Träger Wasser, physiologische Elektrolytlösung, die wäßrige Lösung von ein- oder mehrwertigen Alkoholen wie Glycerin, Polyethylenglycol oder Propylenglykolmethylester oder wäßrige Lösung eines Mono- oder Disaccharides enthalten. Als physiologisch verträglicher flüssiger Träger kann aber auch Wasser oder physiologische Kochsalzlösung in dem Mittel enthalten sein. Ferner ist vorgesehen, daß das Mittel Mikropartikel einer Mischung von Butylstearat und Galaktose in Wasser enthält sowie eine Mischung von Sojaölsaccharoseglycerid und Galaktose in Wasser oder Polyethylenglycolsorbitanmonostearat und Galaktose in physiologischer Kochsalzlösung enthält.

Überraschenderweise wurde weiter gefunden, daß ein erfindungsgemäßes Mittel, welches Mikropartikel von Maltose, Dextrose, Lactose oder Galaktose und Gasbläschen in einem flüssigen Träger enthält, der Wasser, eine physiologische Elektrolytlösung wie 0,9 %ige wäßrige Natriumchloridlösung, Ringer-Lösung oder Tyrode-Lösung oder eine wäßrige Lösung von Maltose, Dextrose, Lactose oder Galaktose ist, ohne Zusatz von viskositätserhöhenden Stoffen wie beispielsweise Propylenglykol eine präzise und gut reproduzierbare Blutdruckmessung blutdurchströmter Organe wie Herz und Adern ermöglicht.

Derartige Mittel, die erfindungsgemäß als Mittel zur Blutdruckmessung verwendet werden können, werden in der EP-OS 131 540 beschrieben.

Dabei kann dieses Mittel Mikropartikel aus Lactose in bis zu 25 %iger (Gewichtsprozent) wäßriger Lactose-Lösung enthalten. Insbesondere können auch Mikropartikel aus Galaktose in bis zu 20 %iger wäßriger Galaktose-Lösung enthalten sein oder Mikropartikel aus Galaktose in Wasser.

Es wurde überraschenderweise festgestellt, daß durch Suspendieren von Mikropartikeln einer festen grenzflächenaktiven Substanz gegebenenfalls in Kombination mit Mikropartikeln eines nicht-grenzflächenaktiven Feststoffes in einer Trägerflüssigkeit Mikrobläschen in Form eines Ensembles erhalten werden, die nach Injektion in eine periphäre Vene Druckmessung in der arteriellen linken Herzkammer ermöglichen.

Derartige Mittel, die erfindungsgemäß als Mittel zur Blutdruckmessung verwendet werden können, werden in EP-OS 122 624 beschrieben.

Bei diesem Mittel sind als grenzflächenaktive Substanz für die Herstellung der Mikropartikel alle Stoffe geeignet, die in den angewandten Mengen physiologisch verträglich sind, d. h. die eine geringe Toxizität besitzen und/oder biologisch abbaubar sind und deren Schmelzpunkt größer als Raumtemperatur ist.

Insbesondere geeignet sind Lecithine, Lecithinfraktionen und deren Abwandlungsprodukte, Polyoxyethylenfettsäureester wie Polyoxyethylenfettalkoholäther, polyoxyethylierte Sorbitanfettsäureester, Glycerin-polyethylenglykoloxystearat, Glycerinpolyethylenglykolrhizinoleat, ethoxylierte Sojasterine, ethoxylierte Rizinusöle und deren hydrierte Derivate, Cholesterol, Polyoxethylenfettsäurestearate und Polyoxyethylenpolyoxypropylen-Polymere mit dem Molgewicht von 6800-8975, 13300 und 16250, Saccharoseester wie Zuckerester, beispielsweise Saccharosedipalmitat und Saccharosemonolaurat oder Saccharosegclyceride sowie Xyloglyceride, gesättigte oder ungesättigte (C₄-C₂₀)-Fettalkohole oder (C₄-C₂₀)-Fettsäuren oder deren Metallsalze, Polyoxyethylenfettsäureester, Mono-, Di- und Triglyceride, Sorbitanfettsäureester, Fettsäureester der Saccharose oder Fettsäureester wie Butylstearat und Ascorbylpalmitat, wobei Calciumstearat, die Saccharoseester der Laurinsäure, der Stearinsäure und der Palmitinsäure sowie Ascorbylpalmitat bevorzugt sind.

Als grenzflächenaktive Substanzen sind solche besonders gut geeignet, die in der Trägerflüssigkeit relativ schwer löslich und im Blut relativ leicht löslich sind. Durch diesen Sprung im Auflöseverhalten der grenzflächenaktiven Substanzen kann die Auflösung des festen Materials, das reich an eingeschlossener Luft ist, gesteuert werden.

Die grenzflächenaktive Substanz wird in eine Konzentration von 0,01 bis 5 Gewichtsprozent, vorzugsweise von 0,04 bis 0,5 Gewichtsprozent verwendet.

Falls erwünscht, können die Mikropartikel der grenzflächenaktiven Substanz mit Mikropartikeln eines physiologisch verträglichen kristallinen Feststoffes kombiniert werden. Man kann dafür organische oder anorganische Stoffe verwenden, zum Beispiel Salze wie Natriumchlorid, Natriumcitrat, Natriumacetat oder Natriumtartrat, Monosaccharide wie Glucose, Fructose oder Galaktose, Disaccharide wie Saccharose, Lactose oder Maltose, Pentosen wie Arabinose, Xylose oder Ribose oder Cyclodextrine wie α-, β-oder γ-Cyclodextrin, wobei Galaktose, Lactose und α-Cyclodextrin bevorzugt sind. Sie sind in einer Konzentration von 5 bis 50 Gewichtsprozent, vorzugsweise von 9 bis 40 Gewichtsprozent, im erfindungsgemäßen Mittel enthalten.

Es liegt ferner im Rahmen der Erfindung, daß ein Mittel zur Blutdruckmessung eine flüssige Mischung zur Aufnahme und Stabilisierung von mit physiologisch verträglichem Gas gefüllten Gasbläschen ist, bestehend aus der Mischung von 0,01 % bis 10 % eines Tensides oder Tensidgemisches mit einer wäßrigen oder mit wasser mischbaren Trägerflüssigkeit und der Mischung von 0,5 % bis 50 % einer viskositätserhöhenden Substanz oder eines Substanzgemisches in einer wäßrigen oder mit Wasser mischbaren Trägerflüssigkeit, wobei beide Mischungen getrennt oder vereinigt vorliegen.

Derartige Mittel, die erfindungsgemäß als Mittel zur Blutdruckmessung verwendet werden können, werden in der EP-PS 0 077 752 beschrieben.

Als Tenside sind sowohl nichtionogene als auch ionogene Tenside geeignet. Als nichtionogene Tenside seien genannt: Lecithine, Lecithinfraktionen und deren Abwandlungsprodukte, Polyoxyethylenfettsäureester wie Polyoxyethylenfettalkoholäther, polyoxyethylierte Sorbitanfettsäureester, Glycerin-polyethylenglykoloxystearat, Glycerinpolyethylenglykolrhizinoleat, ethoxylierte Sojasterine, ethoxylierte Rizinusöle und deren hydrierte Derivate, Cholesterol, Polyoxyethylenpolyoxypropylen-Polymere, wobei Polyoxyethylenfettsäurestearate und Polyoxyethylenpolyoxypropylen-Polymere mit dem Molgewicht 6800-8975, 13300 und 16250 bevorzugt sind. Sämtliche Prozentangaben beziehen sich auf Gewichtsprozente.

Als ionogene Tenside kommen in Frage: Quarternäre Ammoniumbase, Natriumlaurylsulfat, Natriumdioctylsulfosuccinat.

Die Mittel-Lösung kann dabei 0,01 bis 10 % eines Tensides oder des Gemisches mehrerer Tenside enthalten, wobei der bevorzugte Gehalt 0,5 bis 5 % Tensid oder Tensidgemisch beträgt.

Als viskositätserhöhende Substanzen kommen in Frage Mono- oder Polysaccharide wie Glucose, Lävulose, Galaktose, Lactose, Sorbit, Mannit, Xylit, Saccharose oder Dextrane, Cyclodextrine, Hydroxyethylstärke und Polyole. Als Polyole werden verwendet Glycerin, Polyglykole, Inulin und 1,2-Propandiol. Zur Viskositäterhöhung können weiterhin benutzt werden Proteine, proteinähnliche Stoffe, Aminosäuren oder Blutersatzstoffe wie beispielsweise Plasmaproteine, Gelatine, Oxypolygelatine und Gelatinederivate oder deren Gemische.

Die Konzentration dieser genannten Stoffe in der Lösung kann 0,5 bis 50 % betragen, wobei die Höchstkonzentration auch vom gelösten Stoff abhängt. So können beispielsweise Glucose oder Lactose mit einer Konzentration von 0,5 bis 50 % verwendet werden, wogegen Gelatine eine bevorzugte Konzentration von 0,5 bis 2 % hat. Die Oxypolygelatine wird bevorzugt mit einer Konzentration von 0,5 bis 10 % eingesetzt.

Man kann auch Tenside verwenden, die gleichzeitig viskositätserhöhend wirken wie beispielsweise Polyoxyethylenpolyoxpropylen-Polymere mit dem Molekulargewicht von 4750 bis 16250.

In diesem Fall beträgt die Konzentration der Tenside mit viskositätserhöhender Wirkung 1 % bis 20 %, vorzugsweise 3 % bis 10 %. Das Tensid oder Tensidgemisch wird vorzugsweise in Gegenwart des viskositätserhöhenden Stoffes oder Stoffgemische in einer Trägerflüssigkeit gelöst. Als Trägerflüssigkeit kann Wasser verwendet werden oder wäßrige Lösungen, die physiologisch verträglich sind wie beispielsweise physiologische Elektrolytlösungen wie physiologische Kochsalzlösung, Ringerlösung oder die wäßrigen Lösungen von Natriumchlorid, Kalziumchlorid, Natriumhydrogencarbonat, Natriumcitrat, Natriumazetat oder Natriumtartrat oder Salzlösungen, wie sie üblicherweise als Infusionslösungen verwendet werden.

Ausführungsbeispiele der Erfindung sollen in der folgenden Beschreibung unter Bezugnahme auf die Fig. der Zeichnung erläutert werden.

Es zeigen:

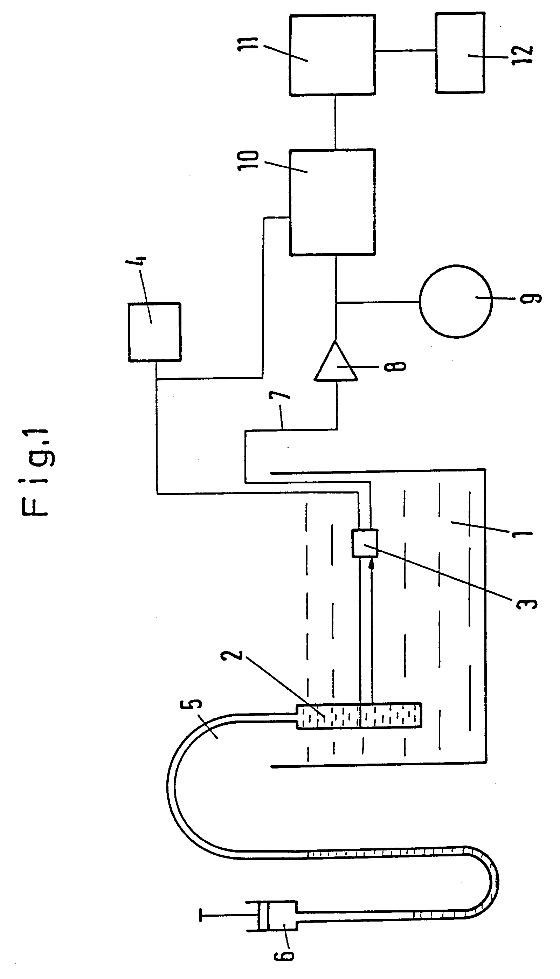

- Fig. 1: eine schematische Darstellung eines Versuchsaufbaues zur Durchführung des Verfahrens,
Wie Fig. 1 zeigt, ist in einem Wasserbad 1 eine quaderförmige, doppelkammerige Plexiglasküvette 2 mit planparalleler Vorder- und Rückwand sowie ein Schallkopf 3 mit Sende- und Empfangsfunktion angeordnet. Der Schallkopf 3 steht mit einem Impulsgeber 4 in Verbindung. Für die Frequenzbereiche 800 KHz bis 2,2 MHz und 2,2 MHz bis 4,2 MHz wurden jeweils verschiedene Schallköpfe 3 mit entsprechend breitbandiger Impulscharakteristik benutzt. Wie dargestellt, erfolgte die Einstrahlung im 90 Winkel zur Vorderwand.

Die Plexiglasküvette 2 steht über eine Druckleitung 5 mit einem Druckgeber 6 in Verbindung.

Der Ausgang des Schallkopfes 3 ist über die Leitung 7 mit einem Verstärker 8 verbunden, dessen Ausgang, wie dargestellt, mit einem Oszillographen 9 und einer Komparatorschaltung 10 in Verbindung steht. Ein weiterer Eingang der Komparatorschaltung 10 ist, wie dargestellt, mit dem Impulsgeber 4 verbunden. Der Ausgang der Komparatorschaltung steht mit einem Laborrechner 11 in Verbindung, dessen Ausgang einen Drucker 12 speist.

Das Rückwandecho bzw. bei hoch konzentrierter Suspension, das vom Ensemble mit den Mikrobläschen zurückgestreute Signal wird verstärkt und oszillographisch sichtbar gemacht, sowie mit Hilfe eines Frequenzanalysators untersucht. Nach A/D-Wandlung wurden die Meßdaten in einem Laborrechner 11 gespeichert, so daß sich die Möglichkeit einer zusätzlichen Auswertung ergibt. Es können z. B. eine digitale Filterung oder eine graphische Darstellung vorgenommen werden.

## Patentansprüche

1. Ultraschall-Manometrieverfahren in einer Flüssigkeit, bei dem Mikrobläschen in die Flüssigkeit eingebracht, in die Flüssigkeit Ultraschallimpulse eingestrahlt und nach Wechselwirkung mit den Mikrobläschen empfangen und ausgewertet werden, dadurch gekennzeichnet, daß in die Flüssigkeit ein Ensemble, bestehend aus
a) den Mikrobläschen und Mikropartikeln aus einer Mischung aus einer grenzflächenaktiven Substanz mit einem nichtgrenzflächenaktiven Feststoff in einem flüssigen Träger oder
b) aus einer flüssigen Mischung zur Aufnahme und Stabilisierung von mit physiologisch verträglichem Gas gefüllten Gasbläschen, bestehend aus der Mischung von 0,01 % bis 10 % eines Tensides oder Tensidgemisches mit einer wäßrigen oder mit Wasser mischbaren Trägerflüssigkeit und der Mischung von 0,5 % bis 50 % einer viskositätserhöhenden Substanz oder eines Substanzgemisches in einer wäßrigen oder mit Wasser mischbaren Trägerflüssigkeit,
eingebracht wird, wobei ein Ensemble mit einem standardisierten Spektrum der Resonanzabsorption verwendet mittels der empfangenen Ultraschallimpulse die spektrale Absorption zur Feststellung des Absolutwertes des momentanen Flüssigkeitsdruckes ermittelt wird.

2. Verfahren nach Anspruch 1, dadurch gekennzeichnet, daß Ultraschallimpulse mit einem vorbestimmten Frequenzbereich eingestrahlt werden und daß Frequenzbereiche registriert und ausgewertet werden, die außerhalb des Frequenzbereiches der eingestrahlten Ultraschallimpulse liegen.

3. Verfahren nach Anspruch 2, dadurch gekennzeichnet, daß Ultraschallimpulse eingestrahlt werden, deren Frequenzen in einem vorbestimmten Verhältnis, insbesondere einem n-ten harmonischen oder subharmonischen, zu den Resonanzfrequenzen des Ensembles stehen.

4. Verfahren nach einem der Ansprüche 1 - 3, dadurch gekennzeichnet, daß Signalspektren im Frequenzbereich oberhalb der Eigenfrequenzen des Ensembles registriert und ausgewertet werden.

5. Verfahren nach einem der Ansprüche 1 - 4, dadurch gekennzeichnet, daß Mikrobläschen mit Durchmessern im Bereich von 0,1 bis 500 um verwendet werden.

6. Verfahren nach einem der Ansprüche 1 - 5, dadurch gekennzeichnet, daß Ultraschallimpulse mit Frequenzen im Bereich von 800 KHz bis 5 MHz eingestrahlt werden.

7. Mittel für eine Blutdruckmessung mittels des Ultraschall-Manometrieverfahrens nach einem der Ansprüche 1 - 6, bestehend aus einer Suspension, die die Mikrobläschen und Mikropartikel, bestehend aus einer Mischung aus einer halbfesten oder flüssigen grenzflächenaktiven Substanz mit einem nichtgrenzflächenaktiven Feststoff in einem flüssigen Träger, enthält.

8. Mittel nach Anspruch 7, enthaltend als halbfeste oder flüssige grenzflächenaktive Substanz Lecithine, Polyoxyethylenfettsäureester, Glycerinpolyethylenglykolrizinoleat, Polyoxyethylenpolyoxypropylen-Polymere, Saccharoseester, Xyloglyceride, ungesättigte (C₄-C₂₀)-Fettalkohole, ungesättigte (C₄-C₂₀)-Fettsäuren, Mono-, Di- und Triglyceride, Fettsäureester als Mikropartikel in einer Menge von 0,01 bis 10 Gewichtsprozent.

9. Mittel nach Anspruch 7 und 8, enthaltend als halbfeste oder flüssige grenzflächenaktive Substanz Butylstearat, Sojaölsaccharoseglycerid oder Polyethylenglykol-sorbitanmonostearat in einer Konzentration von 0,01 bis 5 Gewichtsprozent, vorzugsweise 0,04 bis 1 Gewichtsprozent.

10. Mittel nach Anspruch 7, enthaltend als nicht-grenzflächenaktiven Feststoff Cyclodextrine, Monosaccharide, Disaccharide, Trisaccharide, Polyole oder anorganische oder organische Salze mit einer Konzentration von 5 bis 50 Gewichtsprozent.

11. Mittel nach Anspruch 7, enthaltend als nicht-grenzflächenaktiven Feststoff Galaktose, Lactose oder α-Cyclodextrin in einer Konzentration von 5 bis 50 Gewichtsprozent, vorzugsweise von 9 bis 40 Gewichtsprozent.

12. Mittel nach Anspruch 7, enthaltend als flüssigen Träger, der physiologisch verträglich ist, Wasser, physiologische Elektrolytlösung, z. B. physiologische Kochsalzlösung, wäßrige Lösung von ein- oder mehrwertigen Alkoholen wie Glycerin, Polyethylenglykol oder Propylenglykolmethylester oder der wäßrigen Lösung eines Mono- oder Disaccharides.

13. Mittel nach Anspruch 7, enthaltend Mikropartikel einer Mischung aus Butylstearat und Galaktose in Wasser.

14. Mittel nach Anspruch 7, enthaltend Mikropartikel einer Mischung aus Sojaölsaccharoseglycerid und Galaktose in Wasser.

15. Mittel nach Anspruch 7, enthaltend Mikropartikel einer Mischung aus Polyethylenglykolsorbitanmonostearat und Galaktose in physiologischer Kochsalzlösung.

16. Mittel nach Anspruch 7, enthaltend Mikropartikel aus Maltose, Dextrose, Lactose oder Galaktose und Gasbläschen in einem flüssigen Träger, wobei der flüssige Träger aus Wasser, physiologischer Elektrolytlösung wie 0,9 %iger Natriumchloridlösung, Ringer-Lösung oder Tyrode-Lösung oder einer wäßrigen Lösung von Maltose, Dextrose, Lactose oder Galaktose besteht.

17. Mittel nach Anspruch 16, enthaltend Mikropartikel aus Lactose in bis zu 25 %iger (Gewichtsprozent) wäßriger Lactose-Lösung.

18. Mittel nach Anspruch 16, enthaltend Mikropartikel aus Galaktose in Wasser, insbesondere in bis zu 20 %iger wäßriger Galaktose-Lösung.

19. Mittel für eine Blutdruckmessung mittels des Ultraschall-Manometrieverfahrens nach einem der Ansprüche 1 - 6, enthaltend Mikrobläschen und Mikropartikel einer festen, grenzflächenaktiven Substanz in Kombination mit Mikropartikeln eines nicht-grenzflächenaktiven Feststoffes in einem flüssigen Träger.

20. Mittel nach Anspruch 19, enthaltend als feste grenzflächenaktive Substanz Lecithine, Polyoxyethylenfettsäureester, Glycerinpolyethylenglykolrizinoleat, Cholesterol, Polyoxyethylenpolyoxypropylen-Polymere, Saccharoseester, Xyloglyceride, gesättigte oder ungesättigte (C₄C₂₀)-Fettalkohole, gesättigte oder ungesättigte (C₄-C₂₀)-Fettsäuren oder deren Metallsalze, Mono-, Di- und Triglyceride, Fettsäureester als Mikropartikel in einer Menge von 0,01 bis 10 Gewichtsprozent.

21. Mittel nach Anspruch 19, enthaltend Magnesiumstearat, Ascorbylpalmitat, Saccharosemonopalmitat, Saccharosemonostearat oder Saccharosedistearat als feste grenzflächenaktive Substanz in Form von Mikropartikel in einer Konzentration von 0,01 bis 5 Gewichtsprozent, vorzugsweise 0,04 bis 1 Gewichtsprozent.

22. Mittel nach Anspruch 19, enthaltend als gegebenenfalls vorhandene Mikropartikel eines nicht-grenzflächenaktiven Feststoffes Cyclodextrine, Monosaccharide, Disaccharide, Trisaccharide, Polyole oder anorganische oder organische Salze als Mikropartikel mit einer Konzentration von 5 bis 50 Gewichtsprozent.

23. Mittel nach einem der Ansprüche 19 - 22, enthaltend als gegebenenfalls vorhandenen nicht-grenzflächenaktiven Feststoff Galaktose, Lactose oder α-Cyclodextrin als Mikropartikel in einer Konzentration von 5 bis 50 Gewichtsprozent, vorzugsweise von 9 bis 40 Gewichtsprozent.

24. Mittel nach einem der Ansprüche 19 - 23, enthaltend als flüssigen Träger, der physiologisch verträglich ist, Wasser, physiologische Elektrolytlösung, wäßrige Lösung von ein- oder mehrwertigen Alkoholen wie Glycerin, Polyethylenglykol oder Propylenglykolmethylester oder wäßrige Lösung eines Mono- oder Disaccharides.

25. Mittel nach Anspruch 19 und 24, enthaltend als physiologisch verträglichen flüssigen Träger Wasser, physiologische Kochsalzlösung, 10 %ige wäßrige Lactose-Lösung oder 20 %ige wäßrige Galaktose-Lösung.

26. Mittel nach Anspruch 19, enthaltend Mikropartikel von Magnesiumstearat und von Galaktose in einer 20 %igen wäßrigen Galaktose-Lösung.

27. Mittel für eine Blutdruckmessung mittels des Ultraschall-Manometrieverfahrens nach einem der Ansprüche 1 - 6, bestehend aus einer flüssigen Mischung zur Aufnahme und Stabilisierung von mit physiologisch verträglichem Gas gefüllten Gasbläschen, bestehend aus der Mischung von 0,01 % bis 10 % eines Tensids oder Tensidgemisches mit einer wäßrigen oder mit Wasser mischbaren Trägerflüssigkeit und der Mischung von 0,5 % bis 50 % einer viskositätserhöhenden Substanz oder eines Substanzgemisches in einer wäßrigen oder mit Wasser mischbaren Trägerflüssigkeit, wobei beide Mischungen getrennt oder vereinigt vorliegen.

28. Mittel nach Anspruch 27, bestehend aus der Mischung von 0,01 % bis 10 % eines Tensides oder Tensidgemisches in einer wäßrigen oder mit Wasser mischbaren Trägerflüssigkeit, die 0,05 % bis 5 % eines physiologisch verträglichen carbonsauren Salzes enthält und der Mischung von 0,5 % bis 50 % einer viskositätserhöhenden Substanz oder eines Substanzgemisches mit einer wäßrigen oder mit Wasser mischbaren Trägerflüssigkeit, die die dem carbonsauren Salz äquivalente Menge physiologisch verträglicher Säure enthält.

29. Mittel nach Anspruch 27, enthaltend ein nichtionogenes Tensid, vorzugsweise ein Polyoxyethylenpolyoxypropylen-Polymeres.

30. Mittel nach Anspruch 29, enthaltend ein nichtionogenes Tensid, das aus Polyoxyethylenpolyoxypropylen-Polymere mit dem Molekulargewicht 6800 bis 8974 und 16250 oder 13300 besteht.

31. Mittel nach Anspruch 29, enthaltend ein nichtionogenes Tensid, das aus einem Polyoxyethylenfettsäureester oder aus Polyoxyethylenstearaten besteht.

32. Mittel nach Anspruch 27, enthaltend ein ionogenes Tensid, vorzugsweise Natriumlaurylsulfat oder Natriumdioctylsulfosuccinat.

33. Mittel nach Anspruch 27, enthaltend als Trägerflüssigkeit Wasser oder mit Wasser mischbare ein- oder mehrwertige Alkohole, physiologische Elektrolytlösung oder eine Infusionslösung oder deren Gemische.

## Claims

1. Process for ultrasonic pressure measurement in a liquid, in which microbubbles are incorporated into the liquid, and ultrasonic pulses are radiated into the liquid and, after interacting with the microbubbles, are received and evaluated, characterised in that there is incorporated into the liquid a system comprising
a) the microbubbles and microparticles of a mixture of a surface-active substance and a non-surface-active solid in a liquid carrier or
b) a liquid mixture for receiving and stabilising gas bubbles filled with physiologically tolerable gas, comprising a mixture of from 0.01 % to 10 % of a surfactant or surfactant mixture and an aqueous or water-miscible carrier liquid and a mixture of from 0.5 % to 50 % of a viscosity-increasing substance or substance mixture in an aqueous or water-miscible carrier liquid,
a system having a standardised spectrum of resonance absorption being used and, by means of the ultrasonic pulses received, the spectral absorption being ascertained in order to establish the absolute value of the transient liquid pressure.

2. Process according to claim 1, characterised in that ultrasonic pulses having a predetermined frequency range are radiated in and frequency ranges that are outside the frequency range of the ultrasonic pulses radiated in are recorded and evaluated.

3. Process according to claim 2, characterised in that ultrasonic pulses are radiated in, the frequencies of which are in a predetermined ratio, especially in an nth harmonic or subharmonic ratio, to the resonance frequencies of the system.

4. Process according to any one of claims 1 to 3, characterised in that signal spectra in the frequency range above the system's characteristic frequencies are recorded and evaluated.

5. Process according to any one of claims 1 to 4, characterised in that microbubbles having diameters in the range of from 0.1 to 500 µm are used.

6. Process according to any one of claims 1 to 5, characterised in that ultrasonic pulses having frequencies in the range of from 800 KHz to 5 MHz are radiated in.

7. Agent for measuring blood pressure by means of the process for ultrasonic pressure measurement according to any one of claims 1 to 6, comprising a suspension that contains the microbubbles and microparticles comprising a mixture of a semi-solid or liquid surface-active substance and a non-surface-active solid in a liquid carrier.

8. Agent according to claim 7, containing as semi-solid or liquid surface-active substance lecithins, polyoxyethylene fatty acid esters, glycerol polyethylene glycol ricinoleate, polyoxyethylene/polyoxypropylene polymers, saccharose esters, xyloglycerides, unsaturated (C₄-C₂₀)-fatty alcohols, unsaturated (C₄-C₂₀)-fatty acids, mono-, di- and tri-glycerides, fatty acid esters in the form of microparticles in an amount of from 0.01 to 10 % by weight.

9. Agent according to claims 7 and 8, containing as semi-solid or liquid surface-active substance butyl stearate, soya oil saccharose glyceride or polyethylene glycol sorbitan monostearate in a concentration of from 0.01 to 5 % by weight, preferably from 0.04 to 1 % by weight.

10. Agent according to claim 7, containing as non-surface-active solid cyclodextrins, monosaccharides, disaccharides, trisaccharides, polyols or inorganic or organic salts at a concentration of from 5 to 50 % by weight.

11. Agent according to claim 7, containing as non-surface-active solid galactose, lactose or α-cyclodextrin in a concentration of from 5 to 50 % by weight, preferably from 9 to 40 % by weight.

12. Agent according to claim 7, containing as liquid carrier that is physiologically tolerable: water, physiological electrolyte solution, for example physiological common salt solution, an aqueous solution of mono- or poly-hydric alcohols, such as glycerol, polyethylene glycol or propylene glycol methyl ester, or an aqueous solution of a mono- or di-saccharide.

13. Agent according to claim 7, containing microparticles of a mixture of butyl stearate and galactose in water.

14. Agent according to claim 7, containing microparticles of a mixture of soya oil saccharose glyceride and galactose in water.

15. Agent according to claim 7, containing microparticles of a mixture of polyethylene glycol sorbitan monostearate and galactose in physiological common salt solution.

16. Agent according to claim 7, containing microparticles of maltose, dextrose, lactose or galactose and gas bubbles in a liquid carrier, the liquid carrier comprising water, physiological electrolyte solution, such as 0.9 % sodium chloride solution, Ringer's solution or Tyrode's solution or an aqueous solution of maltose, dextrose, lactose or galactose.

17. Agent according to claim 16, containing microparticles of lactose in an up to 25 % (% by weight) aqueous lactose solution.

18. Agent according to claim 16, containing microparticles of galactose in water, especially in an up to 20 % aqueous galactose solution.

19. Agent for measuring blood pressure by means of the process for ultrasonic pressure measurement according to any one of claims 1 to 6, containing microbubbles and microparticles of a solid, surface-active substance in combination with microparticles of a non-surface-active solid in a liquid carrier.

20. Agent according to claim 19, containing as solid surface-active substance lecithins, polyoxyethylene fatty acid esters, glycerol polyethylene glycol ricinoleate, cholesterol, polyoxyethylene/polyoxypropylene polymers, saccharose esters, xyloglycerides, saturated or unsaturated (C₄-C₂₀)-fatty alcohols, saturated or unsaturated (C₄-C₂₀)-fatty acids or the metal salts thereof, mono-, di- and tri-glycerides, fatty acid esters in the form of microparticles in an amount of from 0.01 to 10 % by weight.

21. Agent according to claim 19, containing magnesium stearate, ascorbyl palmitate, saccharose monopalmitate, saccharose monostearate or saccharose distearate as solid surface-active substance in the form of microparticles in a concentration of from 0.01 to 5 % by weight, preferably from 0.04 to 1 % by weight.

22. Agent according to claim 19, containing as optionally present microparticles of a non-surface-active solid cyclodextrins, monosaccharides, disaccharides, trisaccharides, polyols or inorganic or organic salts in the form of microparticles at a concentration of from 5 to 50 % by weight.

23. Agent according to any one of claims 19 to 22, containing as optionally present non-surface-active solid galactose, lactose or α-cyclodextrin in the form of microparticles in a concentration of from 5 to 50 % by weight, preferably from 9 to 40 % by weight.

24. Agent according to any one of claims 19 to 23, containing as liquid carrier that is physiologically tolerable: water, physiological electrolyte solution, an aqueous solution of mono- or poly-hydric alcohols, such as glycerol, polyethylene glycol or propylene glycol methyl ester, or an aqueous solution of a mono- or disaccharide.

25. Agent according to claims 19 and 24, containing as physiologically tolerable liquid carrier water, physiological common salt solution, 10 % aqueous lactose solution or 20 % aqueous galactose solution.

26. Agent according to claim 19, containing microparticles of magnesium stearate and of galactose in a 20 % aqueous galactose solution.

27. Agent for measuring blood pressure by means of the process for ultrasonic pressure measurement according to any one of claims 1 to 6, comprising a liquid mixture for receiving and stabilising gas bubbles filled with physiologically tolerable gas, comprising a mixture of from 0.01 % to 10 % of a surfactant or surfactant mixture and an aqueous or water-miscible carrier liquid and a mixture of from 0.5 % to 50 % of a viscosity-increasing substance or substance mixture in an aqueous or water-miscible carrier liquid, the two mixtures being separate or combined.

28. Agent according to claim 27, comprising a mixture of from 0.01 % to 10 % of a surfactant or surfactant mixture in an aqueous or water-miscible carrier liquid that contains from 0.05 % to 5 % of a physiologically tolerable carboxylic acid salt and a mixture of from 0.5 % to 50 % of a viscosity-increasing substance or substance mixture and an aqueous or water-miscible carrier liquid that contains an amount of physiologically tolerable acid equivalent to the carboxylic acid salt.

29. Agent according to claim 27, containing a non-ionic surfactant, preferably a polyoxyethylene/polyoxypropylene polymer.

30. Agent according to claim 29, containing a non-ionic surfactant that comprises polyoxyethylene/polyoxypropylene polymers having a molecular weight of from 6800 to 8974 and 16250 or 13300.

31. Agent according to claim 29, containing a non-ionic surfactant that comprises a polyoxyethylene fatty acid ester or polyoxyethylene stearates.

32. Agent according to claim 27, containing an ionic surfactant, preferably sodium lauryl sulphate or sodium dioctyl sulphosuccinate.

33. Agent according to claim 27, containing as carrier liquid water or water-miscible mono- or poly-hydric alcohols, physiological electrolyte solution or an infusion solution or mixtures thereof.

## Revendications

1. Procédé manométrique par ultrasons dans un liquide, selon lequel on introduit des microbulles dans le liquide, on fait passer dans le liquide des impulsions d'ultrasons et, après un effet alterné on les reçoit avec les microbulles et on en fait la cotation d'exploitation, procédé caractérisé en ce qu'on introduit dans le liquide un ensemble consistant en
a) les microbulles et des microparticules d'un mélange d'une substance tensio-active et d'un solide non tensio-actif dans un véhicule liquide, ou
b) en un mélange liquide destiné à recevoir et stabiliser des bulles de gaz emplies d'un gaz physiologiquement tolérable, consistant en un mélange de 0,01 à 10 % d'un tensio-actif ou d'un mélange de tensio-actifs avec un véhicule liquide aqueux ou miscible à l'eau et le mélange de 0,5 % à 50 % d'une substance élevant la viscosité ou d'un mélange de substances élevant la viscosité, dans un véhicule liquide aqueux ou miscible à l'eau,
de sorte que l'on utilise un ensemble comportant un spectre normalisé d'absorption de résonance, l'absorption spectrale reçue à l'aide des impulsions d'ultrasons permettant d'établir la valeur absolue de la pression momentanée du liquide.

2. Procédé selon la revendication 1, caractérisé en ce que les impulsions d'ultrasons sont introduites avec un domaine prédéterminé de fréquences et en ce qu'on enregistre et soumet à cotation d'évaluation les domaines de fréquences qui se situent en dehors du domaine de fréquences des impulsions d'ultrasons que l'on introduit dans le liquide.

3. Procédé selon la revendication 2, caractérisé en ce qu'on introduit des impulsions d'ultrasons dont les fréquences se situent dans un rapport prédéterminé, notamment une harmonique d'ordre n ou des sous-harmoniques, par rapport à la fréquence de résonance de l'ensemble.

4. Procédé selon l'une des revendications 1 à 3, caractérisé en ce qu'on enregistre et soumet à cotation d'évaluation des spectres de signaux dans le domaine de fréquences au-delà des fréquences propres de l'ensemble.

5. Procédé selon l'une des revendications 1 à 4, caractérisé en ce qu'on utilise des microbulles dont les diamètres se situent entre 0,1 et 500 µm.

6. Procédé selon l'une des revendications 1 à 5, caractérisé en ce qu'on introduit des impulsions d'ultrasons ayant des fréquences se situant dans le domaine de 800 KHz à 5 MHz.

7. Moyen pour une mesure de la pression sanguine à l'aide du procédé manométrique par ultrasons selon l'une des revendications 1 à 6, consistant en une suspension qui contient les microbulles et des microparticules consistant en un mélange d'une substance tensio-active semi solide ou liquide avec un solide non tensio-actif dans un véhicule liquide.

8. Moyen selon la revendication 7, contenant comme substance tensio-active semi solide ou liquide des lecithines, des esters d'acides gras polyoxyéthyléniques, du polyéthylène glycol ricinoléate de glycérol, des polymères de type polyoxyéthylène-polyoxypropylène, des esters de saccharose, des xyloglycérides, des alcools gras insaturés en C₄ à C₂₀, les acides gras insaturés en C₄ à C₂₀, des mono-, di- et triglycérides, des esters d'acides gras sous forme de microparticules présentes en une quantité de 0,01 à 10 % en poids.

9. Moyen selon les revendications 7 et 8, contenant comme substance tensio-active semi solide ou liquide du stéarate de butyle, du glycéride de saccharose d'huile de soja ou du monostéarate de polyéthylène glycolsorbitanne, en une concentration de 0,01 à 5 % en poids, avantageusement 0,04 à 1 % en poids.

10. Moyen selon la revendication 7, contenant comme solide non tensio-actif des cyclodextrines, des monosaccharides, des disaccharides, des trisaccharides, des polyols ou des sels minéraux ou organiques présents en une concentration de 5 à 50 % en poids.

11. Moyen selon la revendication 7, contenant comme solide non tensio-actif des galactoses, des lactoses ou de l'α-cyclodextrine, présent(e)(s) en une concentration de 5 à 50 % en poids, avantageusement de 9 à 40 % en poids.

12. Moyen selon la revendication 7, contenant comme véhicule liquide qui est physiologiquement compatible, de l'eau, une solution physiologique d'électrolyte(s), par exemple une solution physiologique de sel de cuisine, une solution aqueuse d'alcools monovalents ou plurivalents comme le glycérol, du polyéthylène glycol ou un ester méthylique de propylène glycol ou la solution aqueuse d'un monosaccharide ou d'un disaccharide.

13. Moyen selon la revendication 7, contenant des microparticules d'un mélange de stéarate de butyle et de galactose dans de l'eau.

14. Moyen selon la revendication 7, contenant des microparticules d'un mélange de glycéride de saccharose d'huile de soja et de galactose dans de l'eau.

15. Moyen selon la revendication 7, contenant des microparticules d'un mélange de monostéarate de polyéthylèneglycol-sorbitanne et de galactose dans une solution physiologique de sel de cuisine.

16. Moyen selon la revendication 7, contenant des microparticules de maltose, de dextrose, de lactose ou de galactose et des bulles ou vésicules de gaz dans un véhicule liquide, le véhicule liquide consistant en de l'eau, une solution physiologique d'électrolyte(s) comme une solution à 0,9 % de chlorure de sodium, une solution de Ringer ou une solution de Tyrode ou une solution aqueuse de maltose, de dextrose, de lactose ou de galactose.

17. Moyen selon la revendication 16, contenant des microparticules de lactose dans une solution aqueuse contenant jusqu'à 25 % (pourcentage en poids) de lactose.

18. Moyen selon la revendication 16, contenant des microparticules de galactose dans de l'eau, en particulier dans une solution aqueuse contenant jusqu'à 20 % de galactose.

19. Moyen pour une mesure de pression sanguine à l'aide du procédé manométrique par ultrasons selon l'une des revendications 1 à 6, ce moyen contenant des microbulles et des microparticules d'une substance tensio-active solide en combinaison avec des microparticules d'un solide non tensio-actif dans un véhicule liquide.

20. Moyen selon la revendication 19, contenant comme substance tensio-active solide des lécithines, des esters polyoxyéthyléniques d'acides gras, du polyéthylèneglycol ricinoléate de glycérol, du cholestérol, des polymères de type polyoxyéthylène-polyoxypropylène, un ester de saccharose, des xyloglycérides, des alcools gras en C₄ à C₂₀, saturés ou insaturés, des acides gras en C₄ à C₂₀, saturés ou insaturés, ou leurs sels de métaux, des monoglycérides, des diglycérides et des triglycérides, des esters d'acides gras sous forme de microparticules présentes en une quantité de 0,01 à 10 % en poids.

21. Moyen selon la revendication 19, contenant du stéarate de magnésium, du palmitate d'ascorbyle, du monopalmitate de saccharose, du monostéarate de saccharose ou du distéarate de saccharose comme substance tensio-active solide, sous forme de microparticules présentes en une concentration de 0,01 à 5 % en poids, avantageusement 0,04 à 1 % en poids.

22. Moyen selon la revendication 19, contenant,microparticules éventuellement présentes d'un solide non tensio-actif, des cyclodextrines, des monosaccharides, des disaccharides, des trisaccharides, des polyols, ou des sels minéraux ou organiques sous forme de microparticules présentes en une concentration de 5 à 50 % en poids.

23. Moyen selon l'une des revendications 19 à 22, contenant, comme solide non tensio-actif éventuellement présent, du galactose, du lactose ou de l'α-cyclodextrine sous forme de microparticules présentes en une concentration de 5 à 50 % en poids, avantageusement de 9 à 40 % en poids.

24. Moyen selon l'une des revendications 19 à 23, contenant comme véhicule liquide, qui est physiologiquement tolérable, de l'eau, une solution aqueuse d'électrolyte(s), une solution aqueuse de monoalcools ou de plurialcools comme le glycérol, du polyéthylène glycol ou un ester méthylique de propylène glycol, ou une solution aqueuse d'un monosaccharide ou d'un disaccharide.

25. Moyen selon les revendications 19 et 24, contenant, comme véhicule liquide physiologiquement tolérable de l'eau, une solution physiologique de sel de cuisine, une solution aqueuse contenant 10 % de lactose ou une solution aqueuse contenant 20 % de galactose.

26. Moyen selon la revendication 19, contenant des microparticules de stéarate de magnésium et de galactose dans une solution aqueuse à 20 % de galactose.

27. Moyen pour une mesure de la pression sanguine à l'aide du procédé manométrique par ultrasons selon l'une des revendications 1 à 6, ce moyen consistant en un mélange liquide destiné à recevoir et à stabiliser des bulles de gaz emplies de gaz physiologiquement compatible, consistant en le mélange de 0,01 % à 10 % d'un tensio-actif ou d'un mélange de tensio-actifs avec un véhicule liquide aqueux ou miscible à l'eau, et le mélange de 0,5 % à 50 % d'une substance ou d'un mélange de substances élevant la viscosité dans un véhicule liquide aqueux ou miscible à l'eau, les deux mélanges étant présents séparément ou étant unis.

28. Moyen selon la revendication 27, consistant en le mélange de 0,01 % à 10 % en poids d'un tensio-actif ou d'un mélange de tensio-actifs dans un véhicule liquide aqueux ou miscible à l'eau, qui contient 0,05 % à 5 % d'un sel d'acide carboxylique physiologiquement tolérable et le mélange de 0,5 % à 50 % d'une substance ou d'un mélange de substances élevant la viscosité avec un véhicule liquide aqueux ou miscible à l'eau qui contient la quantité d'acide physiologiquement tolérable qui équivaut au sel d'acide carboxylique.

29. Moyen selon la revendication 27, contenant un tensio-actif non ionogène, avantageusement un polymère de type polyoxyéthylène-polyoxypropylène.

30. Moyen selon la revendication 29, contenant un tensio-actif non ionogène qui consiste en des polymères de polyoxyéthylène-polyoxypropylène ayant un poids moléculaire de 6 800 à 8 974 et de 16 250 ou 13 300.

31. Moyen selon la revendication 29 contenant un tensio-actif non ionogène, qui consiste en un ester d'acide gras polyoxyéthylénique ou en des stéarates polyoxyéthyléniques.

32. Moyen selon la revendication 27, contenant un tensio-actif non ionogène, avantageusement du laurylsulfate de sodium ou du dioctylsulfosuccinate de sodium.

33. Moyen selon la revendication 27, contenant, comme véhicule liquide, de l'eau ou des monoalcools ou des plurialcools miscibles à l'eau, une solution physiologique d'électrolytes ou une solution pour infusion ou leurs mélanges.
